# EUROPEAN PATENT SPECIFICATION

(11) **EP 0 845 263 B1**
(45) Date of publication and mention of the grant of the patent: **11.12.2002**
(21) Application number: 97118047.6
(22) Date of filing: 17.10.1997
(51) Int. Cl.: A61K 31/195, A61K 9/48, A61K 47/10, A61P 29/00

(54) **Capsules containing diclofenac or salts thereof in solution**
Kapseln enthaltend Diclofenac oder dessen Salze in Lösung
Capsules contenant du diclofenac ou ses sels en solution

(30) Priority: 20.11.1996 IT BO960595
(43) Date of publication of application: 03.06.1998
(73) Proprietor: ALFA WASSERMANN S.p.A., 65020 Alanno Scalo (Pescara) (IT)
(72) Inventor: Rotini, Leone Gabriele, 40133 Bologna (IT); Marchi, Egidio, 40033 Casalecchio di Reno (Bologna) (IT)
(74) Representative: Kraus, Walter, Dr.

(56) References cited:
- EP-A- 0 136 470
- EP-A- 0 185 374
- EP-A- 0 372 527
- EP-A- 0 595 766
- WO-A-88/02625
- WO-A-94/14423

## Description

### BACKGROUND OF THE INVENTION

The capsules, made by soft or hard gelatine, are an important pharmaceutical form for the oral administration of drugs as they offer guarantee of good preservation and of easy handling.

The limit to their use comes from the dimensions, as they have to be swallowed entire without any risk and from the need of encapsulating the drugs under solid form because many solvents, beginning from water, dissolve the gelatine.

A dissolved drug is often better absorbed and causes less side effects produced by local accumulation of a drug administered under solid form.

These side effects are particularly evident in the non-steroidal antiinflammatory drugs of acidic type, coming from the arylacetic acids, which easily cause ulcers in the gastric wall, ulcers fostered from the high local concentration obtained when these drugs are administered under solid form.

Therefore the problem to be solved is finding solvents that dissolve the drug in a very high way, so as to get very small volumes of solution containing the desired therapeutic amount of drug, and that contemporaneously do not interfere at all with the gelatine of the capsules.

They tried to solve the problem in International Publication WO 8802625 by attempting to increase the solubility of antiinflammatory drugs like ibuprofen, naproxen and indomethacin by adding aqueous solutions of potassium hydroxide to the polyethylene glycol used as solvent. Subsequently the International Publication WO 9414423 took up again the same scheme by using the alkaline ionization coupled with other solvents like polyglycerol oleate and the caprilic/capric esters of the glycerol. The partial ionization made by the hydroxylic ions made easier the solubilization of the antiinflammatory drugs in both cases, but the presence of water and alkalinity produced an unfavourable ambient to the stabilities of both the drug and the gelatine capsule.

These severe drawbacks, which limit the applicability of the above mentioned inventions, are now overcome, as regards the non-steroidal antiinflammatory drug known as diclofenac (INN) and the sodium, potassium and hydroxyethylpyrrolidine salts thereof, by dissolving the drug, both under form of free acid and under salified form, with polyethylene glycols, C₈-C₁₀ saturated polyglycosylated glycerides, diethylene glycol monoethylether and mixtures thereof.

### DESCRIPTION OF THE INVENTION

Soft or hard gelatine capsules containing as active principle therapeutically effective amounts of the non-steroidal antiinflammatory drug diclofenac or of the sodium, potassium or hydroxyethylpyrrolidine salts thereof dissolved in a solvent, or in a mixture of solvents, inert towards gelatine, both soft and hard, and contemporaneously endowed with a high dissolving power in respect of the active principle, are the object of the present invention.

It is necessary that the solubility of the active principle into the solvent, or mixture of solvents, is higher than 5%, preferably between 6% and 50% by weight, so that the size of the capsule which contains the solution can be within limits physiologically acceptable, considering that the therapeutically effective dosages of the active principle are between 25 and 150 mg.

It has now been found that the polyethylene glycols, the C₈-C₁₀ saturated polyglycosylated glycerides, the diethylene glycol monoethylether and the mixtures thereof are inert towards the gelatine capsules, both soft and hard, and contemporaneously they dissolve the active principle in an adequate manner, between 6% and 50% by weight.

The invention Is carried out by dissolving in a dissolver equipped with a Ultra-Turax homogenizer the active principle into the solvent, or mixture of solvents, at a concentration by weight between 6% and 50% and by sharing the resulting solution into hard or soft gelatine capsules.

The examples below reported are to be considered as a further illustration of the invention and not as an its limitation.

### EXAMPLE 1

### Soft gelatine capsules containing sodium diclofenac

250 Grams of sodium diclofenac are dissolved into a dissolver equipped with a Ultra-Turax homogenizer in 800 g of polyethylene glycol 400 and the solution is shared into 10,000 soft gelatine capsules each containing 25 mg of active principle.

### EXAMPLE 2

### Soft gelatine capsules containing sodium diclofenac

The test described in example 1 is repeated by using 500 g of sodium diclofenac and 2000 g of diethylene glycol monoethylether and obtaining 10,000 soft gelatine capsules each containing 50 mg of active principle.

### EXAMPLE 3

### Hard gelatins capsules containing potassium diclofenac

500 Grams of potassium diclofenac are dissolved into a dissolver equipped with a Ultra-Turax homogenizer in 1600 g of C₈-C₁₀ saturated polyglycosylated glyceride. The resulting solution is shared into 10,000 hard gelatine capsules which are sealed. Each capsule contains 50 mg of active principle.

### EXAMPLE 4

### Hard gelatine capsules containing sodium diclofenac

The test described in example 3 is repeated by using 500 g of sodium diclofenac and 2000 g of C₈-C₁₀ saturated polyglycosylated glyceride obtaining 10,000 hard gelatine capsules each containing 50 mg of active principle.

### EXAMPLE 5

### Hard gelatine capsules containing diclofenac

The test described in example 3 is repeated by using 250 g of diclofenac and 2250 g of C₈-C₁₀ saturated polyglycosylated glyceride obtaining 10,000 hard gelatine capsules each containing 25 mg of active principle.

### EXAMPLE 6

### Hard gelatine capsules containing the hydroxyethylpyrrolidine salt of the diclofenac

The test described in example 3 is repeated by using 325 g of the hydroxyethylpyrrolidine salt of diclofenac and 2675 g of C₈-C₁₀ saturated polyglycosylated glyceride obtaining 10,000 hard gelatine capsules each containing 32.5 mg of active principle.

### EXAMPLE 7

### Hard gelatine capsules containing sodium diclofenac

The test described in example 3 is repeated by using 500 g of sodium diclofenac and a mixture of solvents made by 700 g of C₈-C₁₀ saturated polyglycosylated glycerides, 700 g of diethylene glycol monoethylether and 700 g of polyethylene glycol 400 obtaining 10,000 hard gelatine capsules each containing 50 mg of active principle.

## Claims

1. A pharmaceutical composition consisting of a solution consisting of therapeutically effective amounts of the non-steroidal antiinflammatory drug diclofenac or of the sodium, potassium or hydroxyethylpyrrolidine salts thereof as active principle and a solvent selected from polyethylene glycols, C₈-C₁₀ saturated polyglycosylated glycerides, diethylene glycol monoethylether and mixtures thereof in soft or hard gelatine capsules.

2. Capsules according to claim 1 **characterized in that** the amount of active principle is between 25 and 150 mg and **in that** its concentration in solution is between 6% and 50% by weight.

## Patentansprüche

1. Pharmazeutische Zubereitung, zusammengesetzt aus einer Lösung, zusammengesetzt aus therapeutisch wirksamen Mengen des nicht-steroidalen antiinflammatorischen Arzneimittels Diclofenac oder der Natrium-, Kalium- oder Hydroxyethylpyrrolidinsalze davon als aktives Prinzip, und einem Lösungsmittel, ausgewählt aus Polyethylenglykolen, gesättigten C₈-C₁₀-polyglykosylierten Glyceriden, Diethylenglykolmonoethylether und den Gemischen davon, in weichen oder harten Gelatinekapseln.

2. Kapseln nach Anspruch 1, **dadurch gekennzeichnet, dass** die Menge an aktivem Prinzip zwischen 25 und 150 mg liegt und dass seine Konzentration in Lösung zwischen 6 und 50 Gew.-% liegt.

## Revendications

1. Composition pharmaceutique consistant en une solution comprenant des quantités efficaces du point de vue thérapeutique de diclofénac, un médicament anti-inflammatoire non stéroïdien ou de ses sels de sodium, de potassium ou d'hydroxyéthylpyrrolidine en tant que principe actif, et un solvant choisi parmi des polyéthylène glycols, des glycérides polyglycosylés saturés en C₈₋₁₀, l'éther monoéthylique de diéthylène glycol et leurs mélanges, dans des gélules de gélatine tendre ou dure.

2. Gélules suivant la revendication 1, **caractérisées en ce que** la quantité de principe actif est comprise entre 25 et 150 mg et que sa concentration en solution est comprise entre 6% et 50% en poids.
